# EUROPÄISCHE PATENTSCHRIFT

(11) **EP 1 043 914 B1**
(45) Veröffentlichungstag und Bekanntmachung des Hinweises auf die Patenterteilung: **16.07.2008**
(21) Anmeldenummer: 99115256.2
(22) Anmeldetag: 02.08.1999
(51) Int. Cl.: H04R 25/00

(54) **Implantierbares Hörsystem**
Implantable hearing system
Système auditif implantable

(30) Priorität: 08.04.1999 DE 19915846
(43) Veröffentlichungstag der Anmeldung: 11.10.2000
(73) Patentinhaber: Cochlear Limited, Lane Cove, NSW 2066 (AU)
(72) Erfinder: Leysieffer, Hans, Dr.-Ing. Dipl.-Ing., 82024 Taufkirchen (DE)
(74) Vertreter: Schwan, Gerhard

(56) Entgegenhaltungen:
- WO-A-98/26629
- WO-A-99/08480
- US-A- 5 456 691
- US-A- 5 603 726

## Beschreibung

Die vorliegende Erfindung betrifft ein mindestens teilweise implantierbares System zur Rehabilitation einer Hörstörung mit einer Anordnung zum Verarbeiten und/oder Generieren von Signalen, die eine implantierbare Prozessoranordnung mit einer gemäß einem Betriebsprogramm arbeitenden Steuerlogik und eine implantierbare Speicheranordnung zum Speichern von Betriebsparametern aufweist, ferner mit einer drahtlosen Telemetrieeinrichtung zur Übertragung von Daten zwischen dem implantierbaren Teil des Systems und einer externen Einheit sowie einer Energieversorgungsanordnung, welche einzelne Komponenten des Systems mit Strom versorgt.

Unter dem Begriff "Hörstörung" sollen vorliegend Innenohrschäden, Mittelohrschäden, kombinierte Innenohr- und Mittelohrschäden, cochleäre Taubheit, die den Einsatz eines Cochlea-Implantats notwendig macht, ebenso wie retrocochleare Hörstörungen, die den Einsatz eines Hirnstamm-Implantats notwendig machen, verstanden werden, d.h. kurz alles was die Schallaufnahme und/oder die Weiterleitung bis zum Hirnstamm verhindert oder beeinträchtigt. Zu "Hörstörungen" gehören vorliegend ferner zeitweise auftretende oder permanente Ohrgeräusche (Tinnitus).

Die Rehabilitation sensorischer Hörstörungen mit teilimplantierbaren, elektronischen Systemen hat in den letzten Jahren einen bedeutenden Stellenwert erhalten. Insbesondere gilt dies für den Patientenkreis, bei dem das Gehör durch Unfall, Krankheit oder sonstige Einflüsse vollständig ausgefallen oder bereits von Geburt an nicht funktionsfähig ist. Ist in diesen Fällen nur das Innenohr (Cochlea) und nicht die nach zentral führende neuronale Hörbahn betroffen, kann mit elektrischen Reizsignalen der verbliebene Hörnerv stimuliert und somit ein Höreindruck erzeugt werden, der bis zu einem offenen Sprachverständnis führen kann. Bei diesen sogenannten Cochlea Implantaten wird ein Reizelektroden-Array in die Cochlea eingeführt, das von einem elektronischen System angesteuert wird, wobei dieses hermetisch dichte und biokompatibel eingekapselte Elektronikmodul operativ im knöchernen Bereich hinter dem Ohr (Mastoid) eingebettet ist. Das elektronische System enthält jedoch im wesentlichen nur Dekodier- und Treiberschaltungen für die Reizelektroden; die akustische Schallaufnahme, die Wandlung dieses Schallsignals in elektrische Signale und deren weitere Verarbeitung erfolgt grundsätzlich extern in einem sogenannten Sprachprozessor, der außen am Körper getragen wird. Der Sprachprozessor setzt die vorverarbeiteten Signale entsprechend kodiert auf ein hochfrequentes Trägersignal um, das über eine induktive Kopplung durch die geschlossene Haut (transkutan) zu dem Implantat übertragen wird. Das schallaufnehmende Mikrofon befindet sich ausnahmslos außerhalb des Körpers und in den meisten Anwendungen in einem an der Ohrmuschel getragenen Gehäuse eines Hinter-dem-Ohr-Hörgerätes (HdO) und ist mit einem Kabel mit dem Sprachprozessor verbunden. Solche Cochlea-Implantat-Systeme, deren Komponenten und Prinzipien der transkutanen Signalübertragung sind beispielhaft in US-A-5 070 535, US-A-4 441 210, EP-A-0 200 321 und US-A-5 626 629 beschrieben. Verfahren zur Sprachaufbereitung und -kodierung bei Cochlea-Implantaten sind beispielsweise in EP-A-0 823 188, EP-B-0 190 836, US-A-5 597 380, US-A-5 271 397, US-A-5 095 904, US-A-5 601 617 und US-A-5 603 726 angegeben.

Neben der Rehabilitation gehörloser bzw. ertaubter Patienten mit Cochlea-Implantaten existieren seit geraumer Zeit Ansätze, Patienten mit einer sensorineuralen Hörstörung, die operativ nicht behebbar ist, mit teil- bzw. vollimplantierbaren Hörgeräten eine bessere Rehabilitation als mit konventionellen Hörgeräten zu bieten. Das Prinzip besteht in den überwiegenden Ausführungsformen darin, ein Ossikel des Mittelohres oder das Innenohr direkt über einen mechanischen bzw. hydromechanischen Reiz zu stimulieren und nicht über das verstärkte akustische Signal eines konventionellen Hörgerätes, bei dem das verstärkte Schallsignal dem äußeren Gehörgang zugeführt wird. Der aktorische Stimulus dieser elektromechanischen Systeme wird mit verschiedenen physikalischen Wandlerprinzipien realisiert wie z.B. durch elektromagnetische und piezoelektrische Systeme. Der Vorteil dieser Verfahren wird hauptsächlich in der gegenüber konventionellen Hörgeräten verbesserten Klangqualität und bei vollimplantierten Systemen in der Unsichtbarkeit der Hörprothese gesehen. Solche teil- und vollimplantierbaren elektromechanischen Hörgeräte sind beispielhaft von Yanigahara et al. in Arch Otolaryngol Head Neck, Surg-Vol 113, August 1987, S. 869-872, Suzuki et al. in Advances in Audiology, Vol. 4, Karger Basel, 1988, Leysieffer et al. in HNO Vol. 46, 1998, S. 853-863, Zenner et al. in HNO Vol. 46, 1998, S. 844-852 und in zahlreichen Patentdokumenten beschrieben, insbesondere in der älteren EP-Patentanmeldung 98 100 918.6, sowie in EP-B-0 499 940, US-A-5 411 467, EP-B-0 400 630, US-A-3 764 748, US-A-4 352 960, US-A-5 015 224, US-A-5 015 225, US-A-3 557 775, US-A-3 712 962, US-A-4 729 366, US-A-4 988 333, EP-B-0 263 254, US-A-5 814 095, US-A-4 850 962, WO-A-98/36711, WO-A-98/06237, US-A-5 859 916, WO-A-98/03035, WO-A-99/08481, WO-A-99/08475, WO-A-99/07436 und in WO-A-97/18689.

WO-A-98/26629 offenbart ein mindestens teilweise implantierbares System zur Rehabilitierung einer Hörstörung, bei welchem eine elektronische Steuerlogik programmiert werden kann. Die Patentschrift US 5,456,691 beschreibt ein implantierbares System, bei welchem mittels einer Telemetrieeinrichtung Funktionsmodule in den Speicher des Systems übertragen werden können.

Viele Patienten mit einem Innenohrschaden leiden zusätzlich unter zeitweise auftretenden oder permanenten Ohrgeräuschen (Tinnitus), die operativ nicht behebbar sind und gegen die bis heute keine medikamentösen Behandlungsformen existieren. Daher sind sogenannte Tinnitus-Maskierer erhältlich; dies sind kleine, batteriebetriebene Geräte, die ähnlich einem Hörgerät hinter oder im Ohr getragen werden und durch artifizielle Schalle, die über einen z.B. Hörgeräte-Lautsprecher in den Gehörgang abgestrahlt werden, den Tinnitus auf psychoakustisch wirkende Weise verdecken ("maskieren") und das störende Ohrgeräusch so möglichst unter die Wahrnehrnungsschwelle absenken. Die artifiziellen Schalle sind häufig Schmalbandgeräusche (z.B. Terzrauschen), die in ihrer spektralen Lage und ihrem Lautstärkepegel über ein Programmiergerät einstellbar sind, um eine möglichst optimale Anpassung an die individuelle Ohrgeräuschsituation zu ermöglichen. Darüber hinaus existiert seit kurzem die sogenannte "Retraining-Methode", wobei durch die Kombination eines mentalen Trainingsprogrammes und die Darbietung eines breitbandigen Schalles (Rauschen) nahe der Ruhehörschwelle die Wahrnehmbarkeit des Tinnitus ebenfalls weitgehend unterdrückt werden soll (Zeitschrift "Hörakustik" 2/97, Seiten 26 und 27). Die hier verwendeten Geräte werden auch als "Noiser" bezeichnet.

Bei beiden oben genannten Methoden zur apparativen Therapie des Tinnitus sind hörgeräteähnliche, technische Geräte außen am Körper im Ohrbereich sichtbar mitzuführen, die den Träger stigmatisieren und daher nicht gerne getragen werden.

In US-A-5 795 287 ist ein implantierbarer Tinnitusmaskierer mit "Direktantrieb" ("direct drive") des Mittelohres z.B. über einen an die Ossikelkette angekoppelten elektromechanischen Wandler beschrieben. Dieser direkt gekoppelte Wandler kann vorzugsweise ein sogenannter "Floating Mass Transducer" (FMT) sein. Dieser FMT entspricht dem Wandler für implantierbare Hörgeräte, der in US-A-5 624 376 beschrieben ist.

Es wurden auch implantierbare Systeme zur Behandlung eines Tinnitus durch Maskierung und/oder Noiserfunktionen vorgeschlagen, bei denen der signalverarbeitende elektronische Pfad eines teil- oder vollimplantierbaren Hörsystems durch entsprechende elektronische Module so ergänzt wird, daß die zur Tinnitusmaskierung oder zur Noiserfunktion notwendigen Signale in den Signalverarbeitungsweg der Hörgerätefunktion einspeisbar sind und die zugehörigen Signalparameter durch weitere elektronische Maßnahmen individuell an die pathologischen Bedürfnisse anpaßbar sind. Diese Anpaßbarkeit kann dadurch realisiert werden, daß die notwendigen Einstelldaten der Signalerzeugungs- und Einspeiselektronik in demselben physikalischen und logischen Datenspeicherbereich des Implantatsystems abgelegt bzw. programmiert werden und über entsprechende elektronische Mittel die Einspeisung des Maskierer- bzw. Noisersignals in den Audiopfad des Hörimplantats steuern.

Bei allen vorstehend genannten Rehabilitationsgeräten erscheint es heute als sehr sinnvoll, die Systeme so auszulegen, daß sie vollständig implantiert werden können. Solche Hörsysteme bestehen je nach angestrebter Funktion aus drei oder aus vier Funktionseinheiten: einem Sensor (Mikrofon), der den einfallenden Luftschall in ein elektrisches Signal umwandelt, einer elektronischen Signalverarbeitungs-, Verstärkungsund Implantatsteuerungseinheit, einem elektromechanischen bzw. implantierbaren elektroakustischen Wandler, der die verstärkten und vorverarbeiteten Sensorsignale in mechanische bzw. akustische Schwingungen umwandelt und diese über geeignete Koppelmechanismen dem geschädigten Mittel- und/oder Innenohr zuführt oder einer cochleären Reizelektrode bei Cochlea-Implantaten sowie einem elektrischen Energieversorgungssystem, das die genannten Module versorgt. Weiterhin kann eine externe Einheit vorgesehen sein, die dem Implantat elektrische Nachladeenergie zur Verfügung stellt, wenn die implantatseitige Energieversorgungseinheit eine nachladbare (Sekundär-)Batterie enthält. Besonders vorteilhafte Vorrichtungen und Verfahren zum Laden von nachladbaren Implantatbatterien sind in der älteren EP-Patentanmeldung 98 121 498.4 sowie in EP-B-0 499 939 beschrieben. Zweckmäßig ist auch eine Telemetrieeinheit vorzusehen, mit der patientenspezifische, audiologische Daten drahtlos bidirektional übertragen bzw. im Implantat programmiert und damit dauerhaft gespeichert werden können, wie es von Leysieffer et al. in HNO Vol. 46, 1998, S. 853-863 beschrieben wurde.

Grundsätzlich sind bei allen genannten, mindestens teilweise implantierbaren Systemen die (Audio-)Signalverarbeitung bzw. Signalerzeugung sowie die Module der Implantatsteuerung wie z.B. ein geregeltes Akkumulator-Nachladesystem oder ein Telemetriesystem zur bidirektionalen Übertragung von z.B. veränderlichen, patientenspezifischen Parametern implantatseitig durch fest vorgegebene Hardwareeinheiten realisiert. Dies gilt auch in den Fällen, wenn zur Signalverarbeitung bzw. -erzeugung oder zum Implantatmanagement digitale Signalprozessoren oder Microcontroller bzw. Mikroprozessoren eingesetzt werden, unabhängig davon, ob diese als eine sogenannte ,,hardwired logic", d.h. in "festverdrahteter" Logikarchitektur, realisiert sind oder ob deren Betriebsprogramme in Festwertspeicherbereichen der entsprechenden Prozessoren abgelegt sind. Diese Programme, die zum grundlegenden Betrieb des Implantats sowie zur bestimmungsgemäßen Funktion notwendig und vorgesehen sind, werden im folgenden als Betriebsprogramm bzw. als Betriebssoftware bezeichnet. Diese Betriebssoftware wird bei den im Stand der Technik beschriebenen Implantatsystemen während der Produktion z.B. durch Maskenprogrammierung der Prozessor-Speicherbereiche in das System eingebracht und ist nach der Implantation nicht mehr veränderbar.

Im Gegensatz werden patientenspezifische Daten wie z.B. audiologische Anpaßdaten oder auch veränderbare Implantatsystemparameter (z.B. als Variable in einem der oben genannten Softwareprogramme zur Regelung einer Akkumulatomachladung) vorliegend als Betriebsparameter bezeichnet. Diese Betriebsparameter können gemäß dem oben angegebenen Stand der Technik bei vollimplantierbaren Implantatsystemen nach der Implantation transkutan, d.h., drahtlos durch die geschlossene Haut, in das Implantat übertragen und damit verändert werden.

Soll dagegen die Betriebssoftware verändert werden, weil z.B. aufgrund neuerer wissenschaftlicher Erkenntnisse verbesserte Algorithmen zur Sprachsignalaufbereitung und - verarbeitung bei z.B. vollimplantierten Cochlea-Implantaten oder elektromechanischen Hörsystemen zur Rehabilitation einer Innenohrschwerhörigkeit vorliegen, muß durch einen invasiven, operativen Patienteneingriff das gesamte Implantat bzw. das Implantatmodul, das die entsprechende Signalverarbeitungseinheit enthält, gegen ein neues mit der veränderten Betriebssoftware ausgetauscht werden. Dieser Eingriff birgt erneute medizinische Risiken für den Patienten, ist insbesondere bei der Applikation von Cochlea Implantaten bei Kindern belastend für den Patienten und wirtschaftlich mit hohem Aufwand verbunden. Darüber hinaus ist dieser Systemwechsel insbesondere bei Cochlea Implantaten nur vollständig, d.h. mit Entfernung der Reizelektrode möglich, da bei der heute üblichen hohen Reizkanalzahl eine technisch sehr aufwendige, mehrpolige und lösbare Steckverbindung zum signalverarbeitenden Implantatmodul nicht realisiert wird.

Demgemäß ist es eine Aufgabe der vorliegenden Erfindung, ein mindestens teilweise implantierbares System zur Rehabilitation einer Hörstörung der eingangs genannten Art zu schaffen, bei welchem sich die Signalverarbeitung bzw. -generierung besser als bei den bisher vorgeschlagenen Systemen nach der Implantation anpassen läßt, um einerseits eine Anpassung der Systemfunktionen an patientenspezifische Gegebenheiten, die sich oftmals erst nach Implantation des Systems feststellen lassen, zu ermöglichen und um andererseits beispielsweise neue medizinische und audiologische Erkenntnisse bei der Signalverarbeitung bzw. -generierung des bereits implantierten Systems berücksichtigen zu können.

Diese Aufgabe wird erfindungsgemäß gelöst durch ein mindestens teilweise implantierbares System zur Rehabilitation einer Hörstörung nach Anspruch 1. Auf diese Weise lassen sich nach Implantation des implantierbaren Systems nicht nur Betriebsparameter von der externen Einheit an das implantierte System übermitteln, sondern es kann auch die Betriebssoftware als solche verändert oder auch vollständig ausgetauscht werden.

Die Speicheranordnung zum Speichern von Betriebsparametern und die Speicheranordnung zur Aufnahme und Wiedergabe des Betriebsprogramms können als voneinander unabhängige Speicher implementiert sein; es kann sich jedoch auch um einen einzigen Speicher handeln, in dem sowohl Betriebsparameter als auch Betriebsprogramme abgelegt werden können.

Die erfindungsgemäße Lösung erlaubt eine Anpassung des Systems an Gegebenheiten, die erst nach Implantation des implantierbaren Systems erfaßbar sind. So sind beispielsweise bei einem mindestens teilweise implantierbaren Hörsystem zur Rehabilitation einer monauralen oder binauralen Mittelohr- und/oder Innenohrstörung sowie eines Tinnitus mit elektrischer und/oder mechanischer und/oder akustischer Stimulation des Mittel-, Innenohres oder der höheren, neuronalen Strukturen der Hörbahn die sensorischen (Schallsensor bzw. Mikrofon) und aktorischen (Ausgangsstimulator) biologischen Schnittstellen immer abhängig von den anatomischen, biologischen und neurophysiologischen Gegebenheiten, z.B. von dem individuellen Einheilprozeß. Diese Schnittstellenparameter können auch zeitvariant sein.

So können beispielsweise das Übertragungsverhalten eines implantierten Mikrofons aufgrund von Gewebebelägen, das Übertragungsverhalten eines an die Ossikelkette bzw. direkt an das Innenohr angekoppelten elektromechanischen Wandlers aufgrund unterschiedlicher Ankopplungsqualität oder aber das Übertragungsverhalten einer intracochleären Elektrode aufgrund unterschiedlicher Elektrodenimpedanzen und Elektrodenlage bei einem Cochlea Implantat oder Hirnstamm-Implantat interindividuell und individuell variieren.

Solche Unterschiede der Schnittstellenparameter, die sich bei den aus dem Stand der Technik bekannten Vorrichtungen nicht einmal durch den Austausch des Implantats mindern bzw. eliminieren ließen, können nun bei dem System nach der Erfindung durch Veränderung bzw. Verbesserung der Signalverarbeitung des Implantats optimiert werden.

Bei einem mindestens teilweise implantierbaren Hörsystem mit elektrischer und/oder mechanischer und/oder akustischer Stimulation des Mittel-, Innenohres oder der höheren, neuronalen Strukturen der Hörbahn kann es sinnvoll bzw. notwendig werden, nach Implantation verbesserte Signalverarbeitungsalgorithmen zu implementieren. Unabhängig von der Art der Stimulation (elektrisch, mechanisch, akustisch) sind hier zu nennen:
- Sprachanalyseverfahren (z.B. Optimierung einer Fast-Fourier-Transformation (FFT))
- Statische oder adaptive Störschallerkennungsverfahren
- Statische oder adaptive Störschallunterdrückungsverfahren
- Verfahren zur Optimierung des systeminternen Signal-Rauschabstandes
- Optimierte Signalverarbeitungsstrategien bei progredienter Hörstörung
- Ausgangspegelbegrenzende Verfahren zum Schutz des Patienten bei Implantatfehlfunktionen bzw. externen Fehlprogrammierungen
- Verfahren zur Vorverarbeitung mehrerer Sensor(Mikrofon)signale, insbesondere bei binauraler Positionierung der Sensoren
- Verfahren zur binauralen Verarbeitung zweier oder mehrerer Sensorsignale bei binauraler Sensorpositionierung z.B. Optimierung des räumlichen Hörens bzw. Raumorientierung
- Phasen- bzw. Gruppenlaufzeit-Optimierung bei binauraler Signalverarbeitung
- Verfahren zur optimierten Ansteuerung der Ausgangsstimulatoren, insbesondere bei binauraler Positionierung der Stimulatoren

Erfolgt die Ausgangsstimulation auf mechanische und /oder akustische Weise, so lassen sich mit dem erfindungsgemäßen System auch nach der Implantation die folgenden Signalverarbeitungsalgorithmen implementieren:
- Verfahren zur Rückkopplungsunterdrückung bzw. -minderung
- Verfahren zur Optimierung des Betriebsverhaltens des bzw. der Ausgangswandler (z.B. Frequenz- und Phasengangoptimierung, Verbesserung des Impulsübertragungsverhaltens)
- Sprachsignal-Kompressionsverfahren bei Innenohrschwerhörigkeiten
- Signalverarbeitungsmethoden zur Recruitment-Kompensation bei Innenohrschwerhörigkeiten

Des weiteren ist bei Implantatsystemen mit einer sekundären Energieversorgungseinheit, d.h. einem nachladbaren Akkumulatorsystem, aber auch bei Systemen mit primärer Batterieversorgung davon auszugehen, daß diese elektrischen Energiespeicher mit voranschreitender Technologie immer größere Lebensdauern und damit steigende Verweilzeiten im Patienten ermöglichen. Es ist davon auszugehen, daß die Grundlagen- und Applikationsforschung für Signalverarbeitungsalgorithmen schnelle Fortschritte macht. Die Notwendigkeit oder der Patientenwunsch einer Betriebssoftwareanpassung bzw. -veränderung wird daher voraussichtlich vor Ablauf der Lebensdauer der implantatinternen Energiequelle eintreten. Während bei den bekannten Systemen mit hardwaregebundener Betriebssoftware eine Anpassung der Betriebssoftware einen operativen Implantatwechsel erforderte, erlaubt das vorliegend beschriebene System eine derartige Anpassung der Betriebsprogramme des Implantats auch im bereits implantierten Zustand.

Bevorzugte Ausführungsformen der vorliegenden Erfindung ergeben sich aus den Unteransprüchen.

Insbesondere kann ferner eine Zwischenspeicheranordnung vorgesehen sein, in welcher von der externen Einheit über die Telemetrieeinrichtung übermittelte Daten zwischengespeichert werden können, bevor diese an die Anordnung zum Verarbeiten und/oder Generieren von Signalen weitergeleitet werden. Auf diese Weise läßt sich der Übertragungsvorgang von der externen Einheit zu dem implantierten System abschließen, bevor die über die Telemetrieeinrichtung übermittelten Daten an die Anordnung zum Verarbeiten und/oder Generieren von Signalen weitergeleitet werden.

Des weiteren kann eine Überprüfungslogik vorgesehen sein, um in der Zwischenspeicheranordnung gespeicherte Daten einer Überprüfung zu unterziehen, bevor diese an die Anordnung zum Verarbeiten und/oder Generieren von Signalen weitergeleitet werden.

Die Anordnung zum Verarbeiten und/oder Generieren von Signalen kann in einem Microcontroller implementiert sein, der vorteilhafterweise auch die Überprüfungslogik und die Zwischenspeicheranordnung enthält, wobei dem Microcontroller zweckmäßig eine implantierbare Speicheranordnung zugeordnet ist, und mindestens Teile eines Arbeitsprogramms für diesen Microcontroller durch von der externen Einheit über die Telemetrieeinrichtung übermittelte Daten geändert oder ausgetauscht werden können.

Die Zwischenspeicheranordnung und die Speicheranordnung zum Speichern des Arbeitsprogramms für den Microcontroller können als voneinander unabhängige Speicher implementiert sein; es kann sich jedoch auch um einen einzigen Speicher handeln, in dem sowohl von der externen Einheit übermittelte Daten als auch Arbeitsprogramme für den Microcontroller abgelegt werden können.

In weiterer Ausgestaltung der Erfindung können mindestens zwei Speicherbereiche zur Aufnahme und Wiedergabe des Betriebsprogramms vorgesehen sein. Auch dies trägt, wie die beiden zuvor genannten Maßnahmen zur Fehlersicherheit des Systems bei, indem durch das mehrfache Vorhandensein des Speicherbereichs, welches das bzw. die Betriebsprogramme enthält, beispielsweise nach einer Übertragung von extern oder aber beim Einschalten des Implantats eine Überprüfung der Fehlerfreiheit der Software durchgeführt werden kann.

Analog hierzu kann auch die Zwischenspeicheranordnung mindestens zwei Speicherbereiche zur Aufnahme und Wiedergabe von von der externen Einheit über die Telemetrieeinrichtung übermittelten Daten aufweisen, so daß nach einer Datenübertragung von der externen Einheit noch im Bereich des Zwischenspeichers eine Überprüfung der Fehlerfreiheit der übermittelten Daten vorgenommen werden kann. Die Speicherbereiche können zur beispielsweise komplementären Ablage der von der externen Einheit übermittelten Daten ausgelegt sein. Mindestens einer der Speicherbereiche der Zwischenspeicheranordnung kann aber auch zur Aufnahme nur eines Teils der von der externen Einheit übermittelten Daten ausgelegt sein, wobei in diesem Fall die Überprüfung der Fehlerfreiheit der übermittelten Daten abschnittsweise erfolgt.

Um zu gewährleisten, daß bei Übertragungsfehlern ein erneuter Übertragungsvorgang gestartet werden kann, kann der Prozessoranordnung ferner ein vorprogrammierter, nicht überschreibbarer Festspeicherbereich zugeordnet sein, in welchem die für einen "Minimalbetrieb" des Systems erforderlichen Anweisungen und Parameter gespeichert sind, beispielsweise Anweisungen, die nach einem "Systemabsturz" zumindest einen fehlerfreien Betrieb der Telemetrieeinrichtung zum Empfang eines Betriebsprogramms sowie Anweisungen zum Einspeichern desselben in die Steuerlogik gewährleisten.

Wie bereits erwähnt, ist die Telemetrieeinrichtung in vorteilhafter Weise außer zur Empfang von Betriebsprogrammen von der externen Einheit auch zur Übermittlung von Betriebsparametern zwischen dem implantierbaren Teil des Systems und der externen Einheit ausgelegt, so daß einerseits solche Parameter von einem Arzt, einem Hörgeräteakustiker oder dem Träger des Systems selbst eingestellt werden können (z.B. Lautstärke), so daß andererseits das System aber auch Parameter an die externe Einheit übermitteln kann, beispielsweise um den Status des Systems zu überprüfen.

Handelt es sich bei dem mindestens teilweise implantierbaren System zur Rehabilitation einer Hörstörung um einen Tinnitusmaskierer, einen Noiser oder ein Hörgerät mit Tinnitusmarkierer- oder Noiser-Funktionen, weist das erfindungsgemäße System vorzugsweise eine digitale Anordnung zum Generieren von elektrischen Signalen und eine der digitalen Anordnung nachgeschaltete Anordnung zum Erzeugen von Stimuli, im vorliegenden Fall von Maskierer- oder Noiser-Signalen, basierend auf den von der digitalen Anordnung generierten elektrischen Signalen auf.

Handelt es sich bei dem mindestens teilweise implantierbaren System zur Rehabilitation einer Hörstörung um ein Hörgerät, so sind gemäß einem weiteren Aspekt der vorliegenden Erfindung mindestens ein Schallsensor, eine digitale Anordnung zum Verarbeiten der mittels des mindestens einen Schallsensors erfaßten Schallsignale, und eine Anordnung zum Erzeugen von Stimuli basierend auf der Verarbeitung der mittels des mindestens einen Schallsensors erfaßten Schallsignale vorgesehen.

In Abhängigkeit von der Funktion des Implantats (insbesondere Cochlea-Implantat, Hirnstamm-Implantat, Tinnitusmaskierer, Noiser, Hörgerät mit oder ohne Tinnitusmaskiereroder Noiserfunktion) kann die Anordnung zum Erzeugen von Stimuli ein Reizelektroden-Array zur Applikation von elektrischen Cochlea- oder Hirnstamm-Stimuli, einen oder mehrere elektroakustische Wandler, einen oder mehrere elektromechanische Wandler, bei welchen es sich auch um piezoelektrische Wandler handeln kann, oder aber einen oder mehrere elektromagnetische Wandler umfassen.

In an sich bekannter Weise umfaßt die Energieversorgungsanordnung des Systems nach der Erfindung vorteilhafterweise eine nachladbare elektrochemische Zelle, die von extern, beispielsweise mittels induktiver Kopplung, nachgeladen werden kann.

Die vorliegende Erfindung wird nachstehend unter Bezugnahme auf die beigefügten Zeichnungen beschrieben, wobei
- FIG. 1: ein schematisches Blockschaltbild eines mindestens teilweise implantierbaren Hörsystems zur Rehabilitation einer Mittel- und/oder Innenohrstörung oder zur Behandlung eines Tinnitus darstellt;
- FIG. 2.: ein Blockschaltbild ähnlich FIG. 1 einer technisch vereinfachten Ausführungsform des erfindungsgemäßen Systems zeigt;
- FIG. 3: eine schematische Ansicht eines im Kopf eines Patienten implantierten Hörsystems sowie zugehöriger externer Einheiten zeigt;
- FIG. 4: eine schematische Ansicht eines implantierten als Cochlea-Implantat ausgelegten Hörsystems nach der Erfindung zeigt;
- FIG. 5: eine schematische Ansicht eines im Kopf eines Patienten implantierten binauralen Hörsystems nach der Erfindung zeigt; und
- FIG. 6: eine schematische Ansicht einer abgewandelten Ausführungsform der Anordnung zum Verarbeiten und/oder Generieren von Signalen zeigt.

In FIG. list ein schematisches Blockschaltbild eines mindestens teilweise implantierbaren Hörsystems 1 zur Rehabilitation einer Mittelohr- und/oder Innenohrstörung oder eines Tinnitus mit elektrischer und/oder mechanischer und/oder akustischer Stimulation des Mittel-, Innenohres oder der höheren, neuronalen Strukturen der Hörbahn dargestellt.

Über einen oder mehrere Schallsensoren (Mikrofone) 10a bis 10n wird das externe Schallsignal aufgenommen und in elektrische Signale umgewandelt. Im Falle einer Implantatrealisierung zur ausschließlichen Rehabilitation eines Tinnitus durch Maskierung oder Noiserfunktion ohne zusätzliche Hörgerätefunktion entfallen diese Sensorfünktionen. Die elektrischen Sensorsignale werden an ein Modul 40 geleitet, in welchem das oder die Sensorsignale ausgewählt und vorverarbeitet werden. Diese Vorverarbeitung kann beispielsweise in einer analogen linearen oder nicht-linearen Vorverstärkung und Filterung (z.B. Antialiasing-Filterung) bestehen. Das vorverarbeitete Sensorsignal führt zu einem Analog-Digital-Wandler 130 (A/D). Bei Verwendung einer Mehrzahl von Sensoren kann auch eine entsprechende Mehrzahl von A/D-Wandlern vorgesehen sein. Das bzw. die digitalisierten Sensorsignale werden einem digitalen Signalprozessor 141 (DSP) zugeführt, der die bestimmungsgemäße Funktion des Hörimplantates ausführt, wie z.B. Audiosignalverarbeitung bei einem System für Innenohrschwerhörigkeiten und/oder Signalgenerierung im Fall eines Tinnitusmaskierers oder Noisers. Der DSP 141 enthält einen nicht überschreibbaren Festspeicherbereich S₀, in welchem die für einen "Minimalbetrieb" des Systems erforderlichen Anweisungen und Parameter gespeichert sind, sowie einen Speicherbereich S₁, in dem die Betriebssoftware der bestimmungsgemäßen Funktion bzw. Funktionen des Implantatsystems abgelegt sind. Wie bereits erwähnt kann dieser Speicherbereich auch doppelt vorhanden sein (S₁'). Der wiederholt beschreibbare Programmspeicher zur Aufnahme der Betriebssoftware kann auf EEPROM-Basis oder RAM-Zellen basieren, wobei in diesem Fall dafür gesorgt sollte, daß dieser RAM-Bereich immer durch das implantatinterne Energieversorgungssystem "gepuffert" ist.

Die digitalen Ausgangssignale des DSP 141 werden in einem Digital-Analog-Wandler 150 (D/A) in Analogsignale umgewandelt. Dieser D/A-Wandler kann je nach Implantatfunktion auch mehrfach ausgelegt sein bzw. völlig entfallen, wenn z.B. im Falle eines Hörsystems mit elektromagnetischem Ausgangswandler direkt ein z.B. pulsweitenmoduliertes, serielles digitales Ausgangssignal des DSP 141 direkt an den Ausgangswandler übermittelt wird. Das bzw. die analogen Ausgangssignale des Digital-Analog-Wandlers 150 sind dann zu einer Treibereinheit 80 geführt, die je nach Implantatfunktion den Ausgangsstimulator 20a ansteuert. Sowohl die Treibereinheit 80 wie auch der Ausgangsstimulator 20a können mehrfach ausgeführt sein (20a bis 20n), z.B. im Falle eines Cochlea-Implantats oder Hirnstamm-Implantats mit mehreren elektrischen Reizelektroden als Ausgangsstimulatoren.

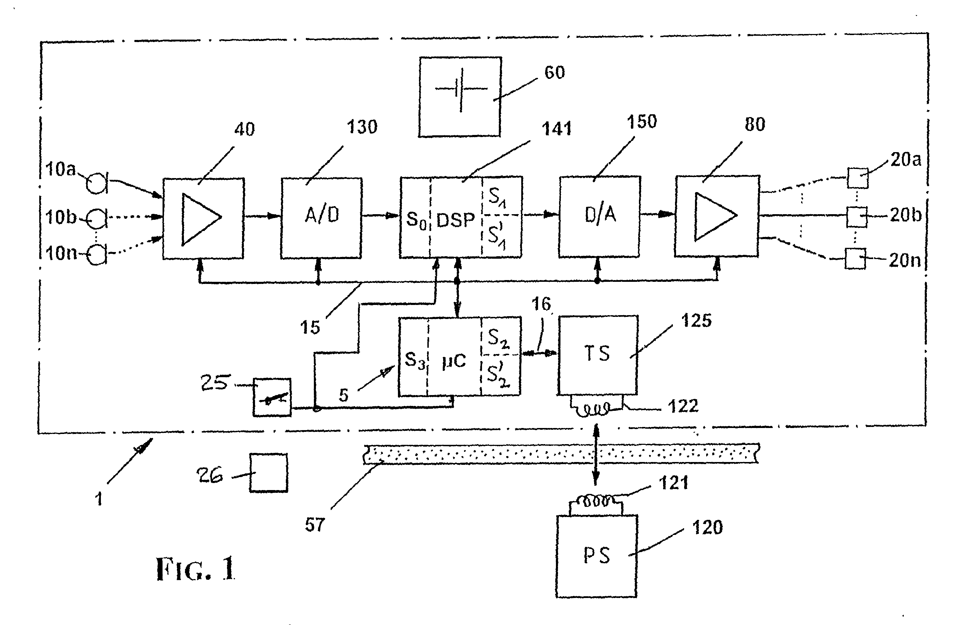

Bei der in FIG. 1 dargestellten Ausführungsform werden die Signalbearbeitungskomponenten 40, 130, 141, 150 und 80 durch einen Microcontroller 5 (µC) mit einem oder zwei zugehörigen Speichern (S₂ bzw. S₂') über einen bidirektionalen Datenbus 15 gesteuert. In dem bzw. den Speicherbereichen S₂ und S₂' können insbesondere die Betriebsoftwareanteile des Implantatmanagementsystems abgelegt sein (z.B. Verwaltungsüberwachungs- und Telemetriefunktionen). In den Speichern S₁ und/oder S₂ können auch von außen veränderliche, patientenspezifische wie z.B. audiologische Anpaßparameter abgelegt sein. Ferner weist der Microcontroller 5 einen wiederholt beschreibbaren Speicher S₃ auf, in welchem ein Arbeitsprogramm für den Microcontroller 5 abgelegt ist.

Der Microcontroller 5 kommuniziert über einen Datenbus 16 mit einem Telemetriesystem 125 (TS). Dieses Telemetriesystem 125 kommuniziert wiederum drahtlos durch die geschlossene Haut 57 über eine in FIG. 1 beispielhaft dargestellte induktive Spulenkopplung (Implantatspule 122 und externe Spule 121) bidirektional mit einem externen Programmiersystem 120 (PS), das vorteilhaft ein Computer mit entsprechender Programmier-, Bearbeitungs-, Darstellungs- und Verwaltungssoftware sein kann. Über diese Telemetrieschnittstelle wird die zu verändernde bzw. ganz auszutauschende Betriebssoftware des Implantatsystems 1 übertragen und zunächst in dem Speicherbereich S₂ des Microcontrollers 5 zwischengespeichert. Der Speicherbereich S₂' kann für eine komplementäre Ablage der von dem externen System übermittelten Daten benutzt werden, und die Koinzidenz der Inhalte der Speicherbereiche S₂ und S₂' kann überprüft werden, bevor der Inhalt des wiederholt beschreibbaren Speicher S₃ geändert oder ausgetauscht wird.

Die Betriebssoftware des mindestens teilweise implantierbaren Hörsystems 1 soll gemäß der vorliegend verwendeten Nomenklatur sowohl die Betriebssoftware des Microcontrollers 5 (zum Beispiel Housekeeping-Funktionen ,wie Energiemanagement oder Telemetriefunktionen) als auch die Betriebssoftware des digitalen Signalprozessors 141 umfassen. So kann z.B. eine einfache Verifikation der Softwareübertragung durch einen Lesevorgang über die Telemetrieschnittstelle durchgeführt werden, bevor die Betriebssoftware oder die entsprechenden Signalverarbeitungsanteile dieser Software in den Programmspeicherbereich S₁ des digitalen Signalprozessors 141 über den Datenbus 15 übertragen werden. Ferner kann auch das Arbeitsprogramm für den Microcontroller 5, das beispielsweise in dem wiederholt beschreibbaren Speicher S₃ eingespeichert ist, über die Telemetrieschnittstelle 125 ganz oder teilweise mit Hilfe der externen Einheit 120 geändert oder ausgetauscht werden.

Alle elektronischen Komponenten des Implantatsystems werden durch eine primäre oder sekundäre Batterie 60 mit elektrischer Betriebsenergie versorgt.

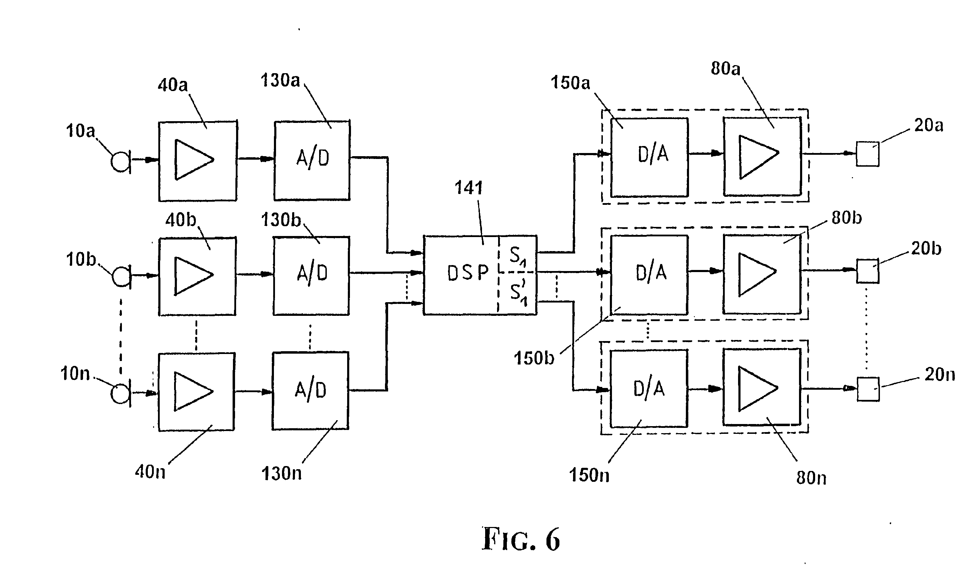

Gemäß FIG. 6 können jedem der Schallsensoren (Mikrofone) 10a bis 10n ein eigenes Vorverarbeitungsmodul 40a bis 40n und ein eigener Analog-Digital-Wandler 130a bis 130n nachgeschaltet sein. In entsprechender Weise sind gemäß FIG. 6 jedem der Ausgangsstimulatoren 20a bis 20n ein eigener Digital-Analog-Wandler 150a bis 150n und eine eigene Treibereinheit 80a bis 80n vorgeschaltet. Je nach bestimmungsgemäßer Implantatfunktion können ausgangsseitig die Digital-Analog-Wandler 150a bis 150n und die Treibereinheiten 80a bis 80n für die Ausgangsstimulatoren 20a bis 20n funktional zusammengefaßt sein, wie dies in FIG. 6 durch die gestrichelten Umrahmungen angedeutet ist. Beispielhaft können im Fall eines Cochlea-Implantats mit mehreren Reizelektroden die von dem digitalen Signalprozessor 141 gelieferten digitalen Ausgangswerte digital programmierbaren Stromquellen zugeführt sein, welche die entsprechenden strom- und zeitmodulierten elektrischen Reizsignale an die Elektroden liefern. Im Fall eines Hörsystems mit einem oder mehreren elekromagnetischen Ausgangswandlern können die Digital-Analog-Wandler 150a bis 150n auch ganz entfallen, wenn die Ausgänge des digitalen Signalprozessors 141 pulsweitenmodulierte serielle Daten liefern und die zugehörige zeitliche Integration durch den oder die Wandler selbst erfolgt.

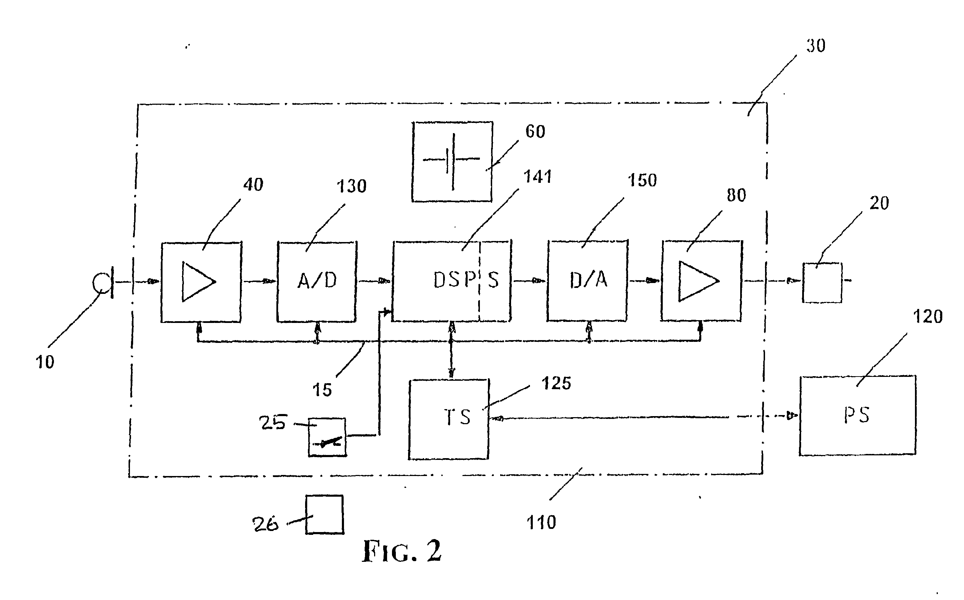

Die technisch vereinfachte Ausführungsform gemäß FIG. 2 unterscheidet sich von derjenigen der FIG. 1 im wesentlichen nur dadurch, daß in dem Modul 30, welches die gesamte Implantatelektronik und den elektrischen Energiespeicher umfaßt, ein Signalprozessor 141 (DSP) vorgesehen ist, der zusätzlich die Funktionen des Microcontrollers 5 gemäß FIG. 1 übernimmt. In FIG. 2 sind ferner jeweils nur ein Sensor 10 und ein Ausgangsstimulator 20 sowie ein Gesamtspeicherbereich S des DSP 141 dargestellt, der sämtliche veränderlichen Betriebs- und Managementsoftwareteile sowie auch patientenspezifische Parameter enthält. In diesem Fall kommuniziert der DSP 141 direkt bidirektional über den Datenbus 15 mit dem Telemetriesystem 125 (TS).

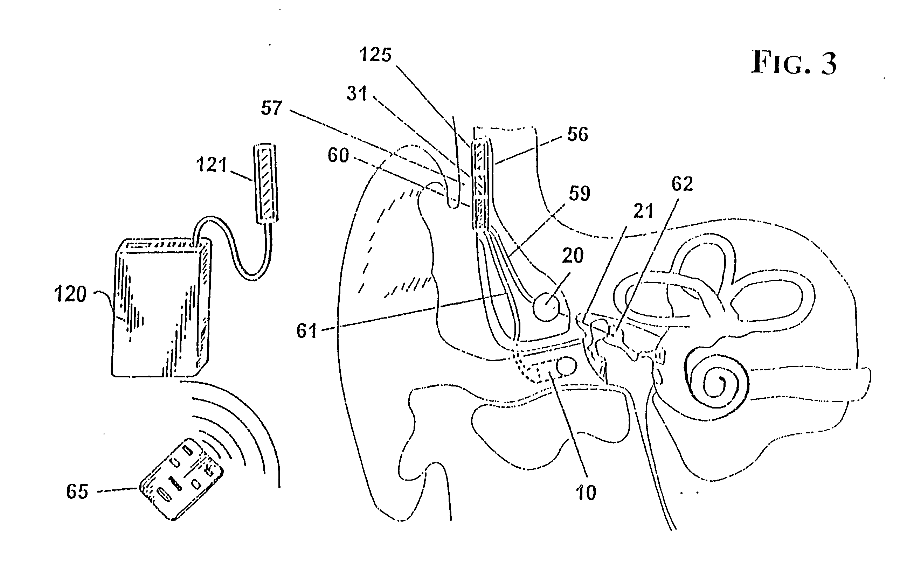

In FIG. 3 ist eine mögliche Ausführungsform eines vollimplantierbaren Hörsystems für Innenohrschwerhörige mit einem Sensor (Mikrofon) und einem elektromechanischen Ausgangswandler mit transkutan veränderbarer Betriebssoftware entsprechend den FIGN. 1, 2 und 6 schematisch dargestellt. Insbesondere ist in einem hermetisch dichten und biokompatiblen Implantatgehäuse 56 ein Elektronikmodul 31 untergebracht, wie es bezugnehmend auf die FIGN. 1, 2 und 6 beschrieben wurde. Weiterhin enthält das Gehäuse 56 eine Batterie 60 zur elektrischen Versorgung des Implantats sowie die Telemetrieeinrichtung 125. Ein in der hinteren Gehörgangswand subkutan implantierter Sensor 10 (Mikrofon) nimmt den Schall auf und wandelt ihn in ein elektrisches Signal um, das über die Implantatleitung 61 dem Elektronikmodul in dem Gehäuse 56 zugeführt wird. Ein besonders vorteilhaftes Mikrofon zur Verwendung bei dem vorliegend beschriebenen System ist in DE-OS 196 38 158 beschrieben.

Das audiologisch bearbeitete und verstärkte Signal wird über die implantierbare Leitung 59 dem elektromechanischen Wandler 20 zugeführt. Dieser Wandler 20 ist im vorliegenden Beispiel als direktgekoppeltes System dargestellt, d.h., die ausgangsseitigen mechanischen Schwingungen des Wandlers 20 werden über ein geeignetes Koppelelement 21 direkt an ein Ossikel der Mittelohrkette gekoppelt, im dargestellten Fall an den Amboß 62. Die dort eingekoppelten Wandlerschwingungen gelangen über die Ossikelkette zum Innenohr und rufen dort den entsprechenden Höreindruck hervor. Geeignete Koppelelemente sind beispielhaft in DE-OS 197 38 587 beschrieben. Vorteilhafte Wandler zum Einsatz bei den vorliegend beschriebenen Systemen sind unter anderem in EP-B-0 499 940 und in den älteren EP-Patentanmeldungen 98 121 495.0 und 98 121 613.8 beschrieben.

Weiterhin ist in FIG. 3 ein externes Programmiersystem 120 dargestellt, mit dem wie beschrieben die zu verändernde oder auszutauschende Betriebssoftware transkutan übertragen werden kann. Dazu dient ein Sende- und Lesekopf mit einer Spule 121, der zur bidirektionalen Datenübermittlung über das Implantat gebracht wird und auf induktivem Weg die Daten transferiert. Ist die Batterie 60 im Implantatgehäuse 56 ein sekundäres, wiederaufladbares Element, kann die implantierbare Einheit auch eine Energieempfangsschaltung zur implantatseitigen Bereitstellung von Nachladeenergie enthalten. Dann enthält das externe System 120 mit der Sendespule 121 auch ein drahtloses Ladegerät. Eine vorteilhafte Ausgestaltung einer implantierbaren Einheit mit einer Empfangsspule ist in der älteren EP-Patentanmeldung 98 121 496.8 beschrieben. In FIG. 3 ist ferner eine portable Fernbedienungseinheit 65 dargestellt, mit welcher der Träger des Systems wesentliche Hörsystemfunktionen einstellen bzw. verändern kann.

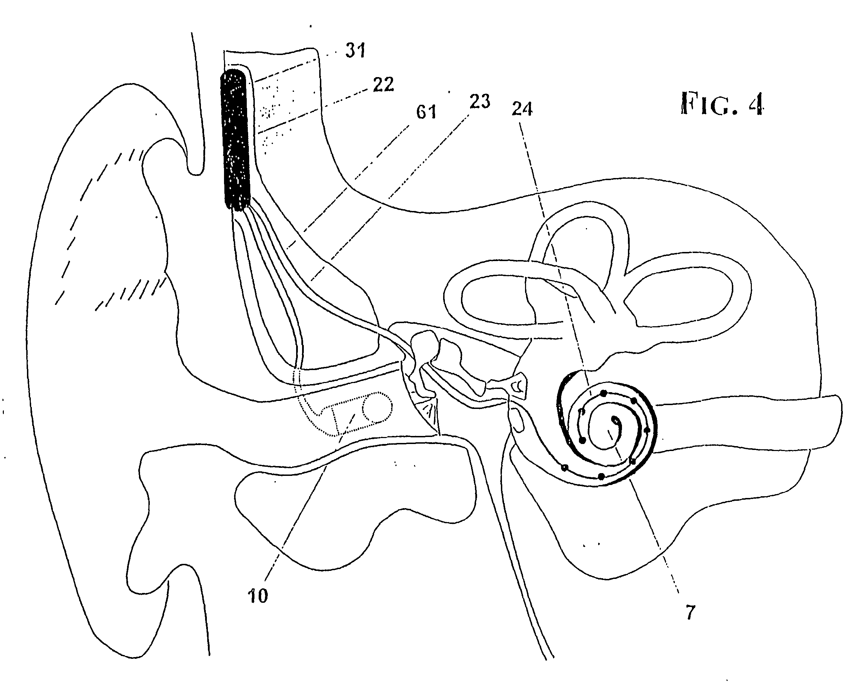

In FIG. 4 ist beispielhaft ein vollständig implantierbares Cochlea-Implantat mit transkutan veränderbarer Betriebssoftware gemäß den FIGN. 1, 2 und 6 dargestellt. Ein Schallsensor 10 (Mikrofon) nimmt externe Schallsignale auf und überträgt diese als elektrische Sensorsignale an ein hermetisch dichtes und biokompatibles Elektronikmodul 31, das in einem artifiziellen Knochenbett 22 auf dem Planum mastoideum fixiert ist. Das Elektronikmodul 31 enthält die Implantatkomponenten gemäß FIG. 1, 2 bzw. 6. Über eine mehrpolige Leitung 23 ist ausgangsseitig eine mehrkanalige elektrische Reizelektrode 24 angeschlossen, die in die Cochlea 7 eingebracht ist. Die zur Übermittlung der Betriebssoftware notwendigen externen Module können identisch wie in FIG. 3 ausgeführt sein und sind daher hier nicht erneut dargestellt.

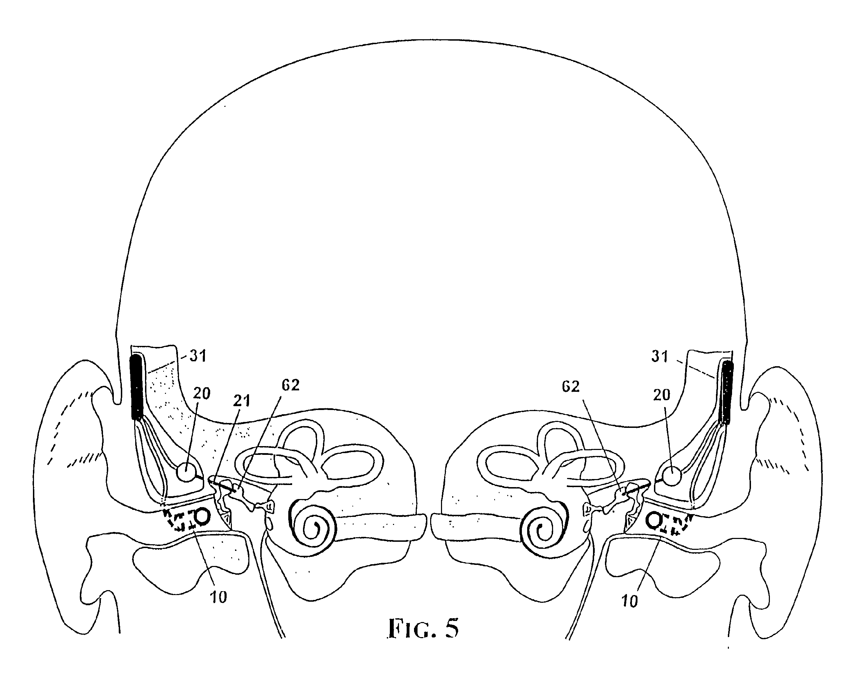

Die vorliegende Erfindung kann nicht nur für eine monaurale, sondern auch für eine binaurale Rehabilitation jeder Art der vorstehend erläuterten Hörstörungen angewendet werden. In FIG. 5 ist beispielhaft ein vollständig implantierbares Hörsystem zur binauralen Versorgung einer beidseitigen Innenohrschwerhörigkeit mit elektromechanischer Stimulation der geschädigten Innenohren dargestellt. Beidseitig ist ein implantierter Schallsensor (Mikrofon) 10 z.B. gemäß FIG. 3 und 4 vorgesehen. Die elektrischen Sensorsignale gelangen in ebenfalls beidseitig auf dem Planum mastoideum positionierte hermetisch dichte und biokompatibel ausgeführte Elektronikmodule 31, welche die Sensorsignale verarbeiten und Signalverarbeitungs- und Energieversorgungskomponenten gemäß FIG. 1, 2 bzw. 6 enthalten können. Die Ausgangssignale führen zu ebenfalls beidseitig implantierten elektromechanischen Wandlern 20. Im dargestellten Beispiel sind diese Wandler 20 über entsprechende Koppelelemente 21 direkt an die Ossikelkette, im gezeigten Ausführungsbeispiel an den Amboß 62, des Mittelohres gekoppelt und übertragen so die mechanischen Schwingungen an die geschädigten Innenohren. Die beiden Elektronikmodule 31 können voneinander unabhängig sein, wobei dann zweckmäßigerweise durch entsprechende Programmierung der einzelnen Module für eine optimale gegenseitige Anpassung gesorgt ist. Die beiden Elektronikmodule 31 können aber auch durch eine elektrische mehrpolige Datenleitung oder drahtlos über eine induktive oder funkbasierte Strecke miteinander verbunden sein, um eine binaurale Signalverarbeitung zu ermöglichen, wie es beispielsweise in WO-A-98/26629 erläutert ist. Hierzu sind in beiden Implantatmodulen entsprechende Schnittstelleneinheiten vorgesehen, welche die galvanische Kopplung bzw. drahtlose Kommunikation realisieren.

Folgende Kombinationsmöglichkeiten sind vorsehbar:
- Beide Elektronikmodule können jeweils einen digitalen Signalprozessor gemäß vorstehender Beschreibung enthalten, wobei die Betriebssoftware beider Prozessoren wie beschrieben transkutan veränderbar ist. Dann sorgt die Verbindung beider Module im wesentlichen für den Datenaustausch zur optimierten binauralen Signalverarbeitung z.B. der Sensorsignale.
- Nur ein Modul enthält den beschriebenen digitalen Signalprozessor, wobei dann die Modulverbindung neben der Sensordatenübertragung zur binauralen Schallanalyse und -verrechnung auch für die Ausgangsignalübermittlung zu dem kontralateralen Wandler sorgt, wobei in dem kontralateralen Modul der elektronische Wandlertreiber untergebracht sein kann. In diesem Fall ist die Betriebssoftware des gesamten binauralen Systems nur in einem Modul abgelegt und wird auch nur dort transkutan über eine nur einseitig vorhandene Telemetrieeinheit von extern verändert. In diesem Fall kann auch die energetische Versorgung des gesamten binauralen Systems in nur einem Elektronikmodul untergebracht sein, wobei die energetische Versorgung des kontralateralen Moduls drahtgebunden oder drahtlos geschieht.

Wie in den Figuren 1 und 2 veranschaulicht ist, kann dem mindestens teilweise implantierbaren Hörsystem 1 beziehungsweise dem Modul 30 ein transkutan betätigbarer Schalter, vorzugsweise ein Zungenschalter 25, zugeordnet sein, der sich dadurch betätigen läßt, daß auf die geschlossene Haut 57 ein Magnet 26 aufgelegt wird. Der Schalter 25 kann beispielsweise in einem Notfall, wie einer Fehlfunktion des Hörsystems, benutzt werden, um die Housekeeping-Funktionen des Microcontrollers 5 zurückzusetzten und/oder den digitalen Signalprozessor 141 zurückzusetzten und/oder die Gehörstimulation bei monauralen oder binauralen Systemen zu sperren. Dabei kann es sich um jede beliebige Art von Reset handeln, der insbesondere das System in seinem Betriebsprogramm zu einem definierten Ablauf zwingt.

Die vorstehend beschriebenen Systeme gestatten Änderungen einer Vielzahl von einzelnen Betriebsprogrammen. So kann es bei manchen Implantatsystemen sinnvoll sein, das Implantatmanagementsystem oder Teile davon auszutauschen. Dies betrifft insbesondere das "Housekeeping-System" sowie Analysefunktionen des Implantates, wie z.B.
- die Ladesteuerung/regelung (bei sekundärer Energieversorgung) sowie die Entladeüberwachung (bei sekundärer und primärer Energieversorgung),
- eine Energieoptimierung des Managementsystems (z.B. programmierbare oder selbstadaptierende Abschaltung von Hardwaremodulen des Implantats),
- eine optimierte Akkuzellenüberwachung bei Systemen mit sekundärer Energieversorgung, insbesondere wie sie vorteilhaft in der älteren EP-Patentanmeldung 98 121 614.6 beschrieben sind,
- eine Optimierung der Telemetrieübertragungssoftware,
- einen Austausch bzw. eine Neugestaltung der Fernbedienungsfunktionen (auch patientenspezifisch),
- implantatinterne Systemmeldungen an den Patienten, beispielsweise über den Ladezustand einer sekundären Energieversorgung, beispielsweise wie es vorteilhaft in der älteren EP Patentanmeldung 98 121 498.4 erläutert ist,
- die telemetrische Übermittlung nach außen von Sensorfunktion(en) bzw. Sensorübertragungsfunktionen, sowie
- audiometrische Implantatfunktionen.

Bei einem vollständig implantierbaren System, das nur als Tinnitusmaskierer bzw- Noiser arbeitet oder einem System, welches sowohl Hörgeräte- als auch Tinnitusmaskierer- bzw. Noiserfunktionen ausführt, können vorteilhaft die Algorithmen zur Signalgenerierung für die Maskierung bzw. Noiserfunktion softwaremäßig austauschbar sein. Dies betrifft grundsätzlich alle Aspekte der Signalgenerierung, z.B. spektrale Lage, Pegel- und Phasenverhältnisse etc., unabhängig davon, ob einzelne Sinussignale, Schmalbandsignale oder Breitbandsignale verwendet werden.

Bei allen der vorgenannten Systeme kann in vorteilhafter Weise ferner
- die Hardwarestruktur des Implantats so ausgelegt sein, daß bei Programmfehlern bzw. externen Störungen oder Speicherschäden oder sonstigen, fehlerauslösenden Vorkommnissen das System in einen für den Patient sicheren, nichtschädigenden Zustand gebracht wird (z.B. durch eine Watchdog-Einheit, intern ausgelösten "Warmstart" oder extern ausgelösten "Kaltstart" (Power-On-Reset) des Implantatsystems),
- die Übertragung der neuen Betriebssoftware mit einem fehlertoleranten oder fehlerkorrigierenden Code erfolgen,
- die Übertragung sowohl von Betriebsparametern als auch von Betriebsprogrammen zwischen dem implantierbaren System und der externen Einheit mit induktiven, infraroten oder elektromagnetischen Verfahren erfolgen,
- die Signalübertragung zwischen dem implantierbaren System und der externen Einheit nach einem "Hand-Shake-Protokoll" erfolgen, und kann
- falls ein EEPROM-Speicherbereich vorgesehen ist, das Implantatsystem ein Hardwaremodul zur Programmierung des EEPROM-Bereiches aufsweisen.

## Patentansprüche

1. Mindestens teilweise implantierbares System zur Rehabilitation einer Hörstörung mit einer Anordnung (40, 40a - 40n, 130, 130a - 130n, 150, 150a - 150n, 80, 80a - 80n; 31) zum Verarbeiten und/oder Generieren von Signalen, die eine implantierbare Prozessoranordnung (141) mit einer gemäß einem Betriebsprogramm arbeitenden Steuerlogik und eine implantierbare Speicheranordnung zum Speichern des Betriebsprogramms und von Betriebsparametern aufweist, ferner mit einer drahtlosen Telemetrieeinrichtung (125) zur Übertragung von Daten zwischen dem implantierbaren Teil des Systems (1, 30, 31) und einer externen Einheit (120) sowie einer Energieversorgungsanordnung (60), welche einzelne Komponenten des Systems mit Strom versorgt, **dadurch gekennzeichnet, dass** der Prozessoranordnung (141) zur Aufnahme und Wiedergabe des Betriebsprogramms und der Betriebsparameter eine wiederholt beschreibbare, implantierbare Speicheranordnung (S; S₁, S₁') zugeordnet ist, und wahlweise Teile des Betriebsprogramms oder das vollständige Betriebsprogramm sowie Betriebsparameter durch von der externen Einheit (120) über die Telemetrieeinrichtung (125) übermittelte Daten geändert oder ausgetauscht werden können, wobei die Telemetrieeinrichtung so ausgebildet ist, dass sie die Daten nach der Implantation des Systems übermitteln kann.

2. System nach Anspruch 1, **dadurch gekennzeichnet, daß** ferner eine Zwischenspeicheranordnung (S₂, S₂') vorgesehen ist, in welcher von der externen Einheit (120) über die Telemetrieeinrichtung (125) übermittelte Daten zwischengespeichert werden können, bevor diese an die Anordnung zum Verarbeiten und/oder Generieren von Signalen weitergeleitet werden.

3. System nach Anspruch 2, **dadurch gekennzeichnet, daß** ferner eine Überprüfungslogik (5) vorgesehen ist, um in der Zwischenspeicheranordnung (S₂, S₂') gespeicherte Daten einer Überprüfung zu unterziehen, bevor diese an die Anordnung (40, 40a - 40n, 130. 130a - 130n, 150, 150a - 150n, 80, 80a - 80n; 31) zum Verarbeiten und/oder Generieren von Signalen weitergeleitet werden.

4. System nach Anspruch 3, **gekennzeichnet durch** einen Microcontroller (5) zum Steuern der Anordnung (40, 40a - 40n, 130, 130a - 130n, 150, 150a - 150n, 80, 80a - 80n; 31) zum Verarbeiten und/oder Generieren von Signalen.

5. System nach den Ansprüchen 3 oder 4, **dadurch gekennzeichnet, daß** die Überprüfungslogik und die Zwischenspeicheranordnung (S₂, S₂') in dem Microcontroller (5) implementiert sind.

6. System nach Anspruch 4 oder 5, **dadurch gekennzeichnet, daß** dem Microcontroller (5) eine implantierbare Speicheranordnung (S₃) zum Speichern eines Arbeitsprogramms für diesen Microcontroller zugeordnet ist, und mindestens Teile des Arbeitsprogramms für den Microcontroller durch von der externen Einheit (120) über die Telemetrieeinrichtung (125) übermittelte Daten geändert oder ausgetauscht werden können.

7. System nach einem der vorhergehenden Ansprüche, **dadurch gekennzeichnet, daß** mindestens zwei Speicherbereiche (S₁, S₁') zur Aufnahme und Wiedergabe mindestens des Betriebsprogramms vorgesehen sind.

8. System nach einem der vorhergehenden Ansprüche, **dadurch gekennzeichnet, daß** die Zwischenspeicheranordnung mindestens zwei Speicherbereiche (S₂, S₂') zur Aufnahme und Wiedergabe von von der externen Einheit (120) über die Telemetrieeinrichtung (125) übermittelten Daten aufweist.

9. System nach einem der vorhergehenden Ansprüche, **dadurch gekennzeichnet, daß** der Prozessoranordnung (141) ferner ein vorprogrammierter, nicht überschreibbarer Festspeicherbereich (S₀) zugeordnet ist.

10. System nach einem der vorhergehenden Ansprüche, **dadurch gekennzeichnet, daß** die Telemetrieeinrichtung (125) zur Übermittlung von Betriebsparametern zwischen dem implantierbaren Teil des Systems (30, 31) und der externen Einheit (120) ausgelegt ist.

11. System nach einem der vorhergehenden Ansprüche, **gekennzeichnet durch** eine digitale Anordnung (141) zum Generieren von elektrischen Signalen und eine der digitalen Anordnung nachgeschaltete Anordnung (20; 20a - 20n; 24) zum Erzeugen von Stimuli basierend auf den von der digitalen Anordnung generierten elektrischen Signalen.

12. System nach einem der vorhergehenden Ansprüche, **gekennzeichnet durch** mindestens einen Schallsensor (10; 10a - 10n), eine digitale Anordnung (141) zum Verarbeiten der mittels des mindestens einen Schallsensors erfaßten Schallsignale, und eine Anordnung (20; 20a - 20n; 24) zum Erzeugen von Stimuli basierend auf der Verarbeitung der mittels des mindestens einen Schallsensors erfaßten Schallsignale.

13. System nach Anspruch 12, **gekennzeichnet durch** eine Vorverarbeitungsanordnung (40, 40a - 40n) zur linearen oder nicht-linearen Verstärkung und/oder zur Filterung von von dem mindestens einen Schallsensor (10; 10a - 10n) kommenden Signalen.

14. System nach Anspruch 13, **dadurch gekennzeichnet, daß** die Vorverarbeitungsanordnung (40, 40a - 40n) einen Antialiasing-Filter umfaßt.

15. System nach einem der Ansprüche 12 bis 14, **dadurch gekennzeichnet, daß** eine Mehrzahl von Schallsensoren (10; 10a - 10n) vorgesehen ist und jedem der Schallsensoren ein Analog-Digital-Wandler (130, 130a-130n) nachgeschaltet ist.

16. System nach einem der Ansprüche 11 bis 15, **gekennzeichnet durch** mindestens einen der Anordnung (20; 20a - 20n; 24) zum Erzeugen von Stimuli vorgeschalteten Digital-Analog-Wandler (150, 150a - 150n).

17. System nach Anspruch 16, **dadurch gekennzeichnet, daß** die Anordnung (20; 20a - 20n; 24) zum Erzeugen von Stimuli eine Mehrzahl von Stimulierzeugern aufweist, denen jeweils ein eigener Digital-Analog-Wandler (150, 150a - 150n) vorgeschaltet ist.

18. System nach einem der Ansprüche 11 bis 17, **gekennzeichnet durch** mindestens eine der Anordnung (20; 20a - 20n; 24) zum Erzeugen von Stimuli vorgeschaltete Treiberanordnung (80, 80a - 80n), welche die von der Anordnung (40, 40a - 40n, 130. 130a - 130n, 150, 150a - 150n; 31) zum Verarbeiten und/oder Generieren von Signalen abgegebenen Signale der Reizform entsprechend aufbereitet.

19. System nach einem der Ansprüche 11 bis 18, **dadurch gekennzeichnet, daß** die Anordnung zum Erzeugen von Stimuli elektrische Stimuli erzeugt und ein Reizelektroden-Array (24) zur Applikation von elektrischen Cochlea- oder Hirnstamm-Stimuli vorgesehen ist.

20. System nach einem der Ansprüche 11 bis 19, **dadurch gekennzeichnet, daß** die Anordnung zum Erzeugen von Stimuli einen oder mehrere elektroakustische Wandler (20, 20a - 20n) umfaßt.

21. System nach einem der Ansprüche 11 bis 20, **dadurch gekennzeichnet, daß** die Anordnung zum Erzeugen von Stimuli einen oder mehrere elektromechanische Wandler (20, 20a - 20n) umfaßt.

22. System nach Anspruch 21, **dadurch gekennzeichnet, daß** die Anordnung zum Erzeugen von Stimuli einen oder mehrere piezoelektrische Wandler (20, 20a - 20n) umfaßt.

23. System nach einem der Ansprüche 11 bis 22, **dadurch gekennzeichnet, daß** die Anordnung zum Erzeugen von Stimuli einen oder mehrere elektromagnetische Wandler (20, 20a - 20n) umfaßt.

24. System nach einem der vorhergehenden Ansprüche, **dadurch gekennzeichnet, daß** die Energieversorgungsanordnung (60) eine nachladbare elektrochemische Zelle aufweist.

25. System nach einem der vorhergehenden Ansprüche, **dadurch gekennzeichnet, daß** es als binaurales System zur Rehabilitation einer Hörstörung beider Ohren ausgelegt ist, das zwei Systemeinheiten (10, 20, 21, 31) aufweist, die jeweils einem der beiden Ohren zugeordnet sind.

26. System nach Anspruch 25, **dadurch gekennzeichnet, daß** die beiden Systemeinheiten (10, 20, 21, 31) einander im wesentlichen gleich sind.

27. System nach Anspruch 25, **dadurch gekennzeichnet, daß** die eine Systemeinheit (10, 20, 21, 31) als Master-Einheit und die andere Systemeinheit (10, 20, 21, 31) als von der Master-Einheit gesteuerte Slave-Einheit ausgelegt ist.

## Claims

1. At least partially implantable system for rehabilitation of a hearing disorder with an arrangement (40, 40a-40n, 130, 130a-130n, 150, 150a-150n, 80, 80a-80n; 31) for processing and/or generating signals, which includes an implantable processor arrangement (141) with control logic, which operates according to an operating program, and an implantable storage arrangement for storage of the operating program and of operating parameters, further comprising a wireless telemetry means (125) for data transmission between the implantable part of the system (1, 30, 31) and an external unit (120) as well as a power supply arrangement (60) which supplies individual components of the system with current, **characterized in that** an implantable and repeatedly rewritable storage arrangement (S; S₁, S₁') is assigned to the processor arrangement (141) for holding and reproducing the operating program and the operating parameters, and parts of the operating program or the entire operating program as well as operating parameters can be selectively modified or replaced by data transmitted from the external unit (120) via the telemetry means (125), wherein the wireless telemetry means is adapted to transmit the data after implantation of the system.

2. System according to claim 1, **characterized in that** further a buffer storage arrangement (S₂, S₂') is provided in which data transmitted from the external unit (120) via the telemetry means (125) are buffered before being relayed to the arrangement for processing and/or generating signals.

3. System according to claim 2, **characterized in that** further a checking logic (5) is provided to check data stored in the buffer storage arrangement (S₂, S₂') before being relayed to the arrangement (40, 40a-40n, 130, 130a-130n, 150, 150a-150n, 80, 80a-80n, 31) for processing and/or generating signals.

4. System according to claim 3, **characterized in that** a microcontroller (5) is provided for controlling the arrangement (40, 40a-40n, 130, 130a-130n, 150, 150a-150n, 80, 80a-80n, 31) for processing and/or generating signals.

5. System according to claim 3, **characterized in that** the checking logic and the buffer storage arrangement (S₂, S₂') are implemented in the microcontroller (5).

6. System according to claim 4 or 5, **characterized in that** an implantable storage arrangement (S₃) for storage of a working program for the microcontroller is assigned to the microcontroller and at least parts of the working program for the microcontroller can be modified or replaced by data transmitted from the external unit (120) via the telemetry means (125).

7. System according to anyone of the preceding claims, **characterized in that** at least two storage areas (S₁, S₁') are provided for holding and reproducing at least the operating program.

8. System according to anyone of the preceding claims, **characterized in that** the buffer storage arrangement includes at least two storage areas (S₂, S₂') for holding and reproducing data transmitted from the external unit (120) via the telemetry means (125).

9. System according to anyone of the preceding claims, **characterized in that** a preprogrammed read-only memory area (S₀), which cannot be overwritten, is assigned to the processor arrangement (141).

10. System according to anyone of the preceding claims, **characterized in that** the telemetry means (125) is adapted to transfer operating parameters between the implantable part of the system (30, 31) and the external unit (120).

11. System according to anyone of the preceding claims, **characterized in that** a digital arrangement (141) is provided for generation of electrical signals and an arrangement (20, 20a-20n, 24) is provided downstream of the digital arrangement for generating stimuli based on the electrical signals generated by the digital arrangement.

12. System according to anyone of the preceding claims, **characterized by** at least one acoustic sensor (10, 10a-10n), a digital arrangement (141) for processing the acoustic signals acquired by means of said at least one acoustic sensor, and an arrangement (20, 20a-20n, 24) for generating stimuli based on the processing of the acoustic signals acquired by the at least one acoustic sensor.

13. System according to claim 12, **characterized by** a pre-processing arrangement (40, 40a-40n) for linear or non-linear amplification and/or for filtering of signals originating from the at least one acoustic sensor (10, 10a-10n).

14. System according to claim 13, **characterized in that** the pre-processing arrangement (40, 40a-40n) comprises an anti-aliasing filter.

15. System according to anyone of claims 12 to 14, **characterized in that** a plurality of acoustic sensors (10, 10a-10n) is provided and an analog digital converter (130, 130a-130n) is connected downstream of each of the plurality of acoustic sensors.

16. System according to anyone of the preceding claims 11 to 15, **characterized in that** at least one digital analog converter (150, 150a-150n) is connected upstream the arrangement (20, 20a-20n; 24) for generating stimuli.

17. System according to claim 16, **characterized in that** the arrangement (20, 20a-20n; 24) for generating stimuli includes a plurality of stimuli producers to which an own digital analog converter (150, 150a-150n) is connected upstream.

18. System according to anyone of claims 11 to 17, **characterized by** at least one driver arrangement (80, 80a-80n) connected upstream of the arrangement (20; 20a-20n; 24) for generating stimuli is provided which, according to a mode of stimulation, processes the signals delivered by the arrangement (40, 40a-40n, 130, 130a-130n, 150a-150n; 31) for processing and/or generating signals.

19. System according to anyone of claims 11 to 18, **characterized in that** the arrangement for generating stimuli produces electrical stimuli and further there is provided an array (24) of stimulation electrodes for application of electrical cochlea or brain stem stimuli.

20. System according to anyone of claims 11 to 19, **characterized in that** the arrangement for generating stimuli comprises one or a plurality of electro-acoustic transducers (20, 20a-20n).

21. System according to anyone of claims 11 to 20, **characterized in that** the arrangement for generating stimuli comprises one or a plurality of electro-mechanical transducers (20, 20a-20n).

22. System according to claim 21, **characterized in that** the arrangement for generating stimuli comprises one or a plurality of piezoelectrical transducers (20, 20a-20n).

23. System according to anyone of claims 11 to 22, **characterized in that** the arrangement for generating stimuli comprises one or a plurality of electromagnetic transducers (20, 20a-20n).

24. System according to anyone of the preceding claims, **characterized in that** the power supply arrangement (60) comprises a rechargeable electrochemical cell.

25. System according to anyone of the preceding claims, **characterized in that** the system is a binaural system for rehabilitation of a hearing disorder of both ears which includes two system units (10, 20, 21, 31), one for each of the ears.

26. System according to claim 25, **characterized in that** the two system units (10, 20, 21, 31) are essentially identical to one another.

27. System according to claim 25, **characterized in that** one system unit (10, 20, 21, 31) is designed as master unit and the other system unit (10, 20, 21, 31) is designed as a slave unit being controlled by the master unit.

## Revendications

1. Système au moins partiellement implantable pour la correction d'un problème auditif avec un dispositif (40, 40a à 40n, 130, 130a à 130n, 150, 150a à 150n, 80, 80a à 80n ; 31) pour le traitement et/ou la génération de signaux, lequel dispositif comporte un dispositif processeur implantable (141) avec une logique de commande travaillant selon un programme d'exploitation et un dispositif mémoire implantable pour la mémorisation du programme d'exploitation et de paramètres d'exploitation, avec en outre un dispositif de télémétrie sans fil (125) pour la transmission de données entre la partie implantable du système (1, 30, 31) et une unité externe (120) ainsi qu'un dispositif d'alimentation en énergie (60) qui alimente en courant différents composants du système, **caractérisé par le fait qu'**un dispositif mémoire implantable (S ; S₁, S₁') dans lequel on peut écrire de manière répétée, est associé au dispositif processeur (141) pour l'enregistrement et la reproduction du programme d'exploitation et des paramètres d'exploitation et que sélectivement des parties du programme d'exploitation ou le programme d'exploitation entier ainsi que des paramètres d'exploitation peuvent être modifiés ou remplacés par des données transmises par l'unité externe (120) par l'intermédiaire du dispositif de télémétrie (125), le dispositif de télémétrie étant conçu de telle sorte qu'il peut transmettre les données après l'implantation du système.

2. Système selon la revendication 1, **caractérisé par le fait qu'**il est prévu en outre un dispositif de mémorisation temporaire (S₂, S₂') dans lequel peuvent être mémorisées temporairement des données transmises par l'unité externe (120) par l'intermédiaire du dispositif de télémétrie (125) avant que lesdites données soient retransmises au dispositif pour le traitement et/ou la génération de signaux.

3. Système selon la revendication 2, **caractérisé par le fait qu'**il est prévu en outre une logique de vérification (5) pour soumettre à une vérification des données mémorisées dans le dispositif de mémorisation temporaire (S₂, S₂') avant que lesdites données soient retransmises au dispositif (40, 40a à 40n, 130, 130a à 130n, 150, 150a à 150n, 80, 80a à 80n ; 31) pour le traitement et/ou la génération de signaux.

4. Système selon la revendication 3, **caractérisé par** un microcontrôleur (5) pour la commande du dispositif (40, 40a à 40n, 130, 130a à 130n, 150, 150a à 150n, 80, 80a à 80n ; 31) pour le traitement et/ou la génération de signaux.

5. Système selon la revendication 4, **caractérisé par le fait que** la logique de vérification et le dispositif de mémorisation temporaire (S₂, S₂') sont implantés dans le microcontrôleur (5).

6. Système selon la revendication 4 ou 5, **caractérisé par le fait qu'**un dispositif de mémorisation implantable (S₃) pour la mémorisation d'un programme de travail destiné au microcontrôleur (5) est associé à ce microcontrôleur et qu'au moins des parties du programme de travail destiné au microcontrôleur peuvent être modifiées ou remplacées par des données transmises par l'unité externe (120) par l'intermédiaire du dispositif de télémétrie (125).

7. Système selon l'une des revendications précédentes, **caractérisé par le fait qu'**il est prévu au moins deux zones de mémoire (S₁, S₁') pour l'enregistrement et la reproduction au moins du programme d'exploitation.

8. Système selon l'une des revendications précédentes, **caractérisé par le fait que** le dispositif de mémorisation temporaire comporte au moins deux zones de mémoire (S₂, S₂') pour l'enregistrement et la reproduction de données transmises par l'unité externe (120) par l'intermédiaire du dispositif de télémétrie (125).

9. Système selon l'une des revendications précédentes, **caractérisé par le fait qu'**une zone de mémoire morte préprogrammée et non réinscriptible (S₀) est associée en outre au dispositif processeur (141).

10. Système selon l'une des revendications précédentes, **caractérisé par le fait que** le dispositif de télémétrie (125) est conçu pour la transmission de paramètres d'exploitation entre la partie implantable du système (30, 31) et l'unité externe (120).

11. Système selon l'une des revendications précédentes, **caractérisé par** un dispositif numérique (141) pour la génération de signaux électriques et par un dispositif (20 ; 20a à 20n ; 24) branché en aval du dispositif numérique pour la production de stimuli en se basant sur les signaux électriques générés par le dispositif numérique.

12. Système selon l'une des revendications précédentes, **caractérisé par** au moins un capteur acoustique (10 ; 10a à 10n), par un dispositif numérique (141) pour le traitement des signaux acoustiques détectés au moyen de l'au moins un capteur acoustique, et par un dispositif (20 ; 20a à 20n ; 24) pour la production de stimuli en se basant sur les signaux acoustiques détectés au moyen de l'au moins un capteur acoustique.

13. Système selon la revendication 12, **caractérisé par** un dispositif de prétraitement (40, 40a à 40n) pour l'amplification linéaire ou non linéaire et/ou pour le filtrage de signaux arrivant de l'au moins un capteur acoustique (10 ; 10a à 10n).

14. Système selon la revendication 13, **caractérisé par le fait que** le dispositif de prétraitement (40 ; 40a à 40n) comprend un filtre antirepliement.

15. Système selon l'une des revendications 12 à 14, **caractérisé par le fait qu'**il est prévu une multiplicité de capteurs acoustiques (10 ; 10a à 10n) et qu'un convertisseur analogique - numérique (130 ; 130a à 130n) est branché en aval de chacun des capteurs acoustiques.

16. Système selon l'une des revendications 11 à 15, **caractérisé par** au moins un convertisseur numérique - analogique (150 ; 150a à 150n) branché en amont du dispositif (20 ; 20a à 20n ; 24) pour la production de stimuli.

17. Système selon la revendication 16, **caractérisé par le fait que** le dispositif (20 ; 20a à 20n ; 24) pour la production de stimuli comporte une multiplicité de producteurs de stimuli en amont desquels est branché à chaque fois un convertisseur numérique - analogique particulier (150 ; 150a à 150n).

18. Système selon l'une des revendications 11 à 17, **caractérisé par** au moins un dispositif d'attaque (80 ; 80a à 80n) qui est branché en amont du dispositif (20 ; 20a à 20n ; 24) pour la production de stimuli et qui prépare conformément à la forme de stimulation les signaux fournis par le dispositif (40, 40a à 40n, 130, 130a à 130n, 150, 150a à 150n ; 31) pour le traitement et/ou la génération de signaux.

19. Système selon l'une des revendications 11 à 18, **caractérisé par le fait que** le dispositif pour la production de stimuli produit des stimuli électriques et qu'une matrice d'électrodes de stimulation (24) est prévue pour l'application de stimuli électriques de cochlée ou de tronc cérébral.

20. Système selon l'une des revendications 11 à 19, **caractérisé par le fait que** le dispositif pour la production de stimuli comprend un ou plusieurs convertisseurs électroacoustiques (20, 20a à 20n).

21. Système selon l'une des revendications 11 à 20, **caractérisé par le fait que** le dispositif pour la production de stimuli comprend un ou plusieurs convertisseurs électromécaniques (20, 20a à 20n).

22. Système selon la revendication 21, **caractérisé par le fait que** le dispositif pour la production de stimuli comprend un ou plusieurs convertisseurs piézoélectriques (20, 20a à 20n).

23. Système selon l'une des revendications 11 à 22, **caractérisé par le fait que** le dispositif pour la production de stimuli comprend un ou plusieurs convertisseurs électromagnétiques (20, 20a à 20n).

24. Système selon l'une des revendications précédentes, **caractérisé par le fait que** le dispositif d'alimentation en énergie (60) comporte une cellule électrochimique rechargeable.

25. Système selon l'une des revendications précédentes, **caractérisé par le fait qu'**il est conçu comme système binauriculaire destiné à la correction d'un problème auditif des deux oreilles et comporte deux unités de système (10, 20, 21, 31) qui sont associées chacune à l'une des deux oreilles.

26. Système selon la revendication 25, **caractérisé par le fait que** les deux unités de système (10, 20, 21, 31) sont globalement identiques l'une à l'autre.

27. Système selon la revendication 25, **caractérisé par le fait que** l'une des unités de système (10, 20, 21, 31) est conçue comme une unité maîtresse et que l'autre unité de système (10, 20, 21, 31) est conçue comme une unité esclave commandée par l'unité maîtresse.
